(19) Europäisches Patentamt
European Patent Office
Office européen des brevets

(11) Publication number: **0 278 505 B1**

(12) **EUROPEAN PATENT SPECIFICATION**

(45) Date of publication of patent specification: **17.06.92** (51) Int. Cl.$^5$: **A61K 7/06**

(21) Application number: **88102000.2**

(22) Date of filing: **11.02.88**

(54) **Hair protection composition and method.**

(30) Priority: **12.02.87 US 13888**

(43) Date of publication of application:
**17.08.88 Bulletin 88/33**

(45) Publication of the grant of the patent:
**17.06.92 Bulletin 92/25**

(84) Designated Contracting States:
**BE CH DE FR GB IT LI**

(56) References cited:
**EP-A- 0 102 534**

**PATENT ABSTRACTS OF JAPAN, vol. 12, no. 472 (C-551)[3319], 9th December 1988; & JP-A-63 192 703 (KAO CORP.) 10-08-1988**

**PATENT ABSTRACTS OF JAPAN, vol. 11, no. 115 (C-415)[2562], 10th April 1987; & JP-A-61 260 008 (SUNSTAR INC.) 18-11-1986**

(73) Proprietor: **ESTEE LAUDER INC.**
**767 Fifth Avenue**
**New York New York 10022(US)**

(72) Inventor: **Smith, Walter P.**
**89 Blackberry Drive**
**Stamford Connecticut 06903(US)**

(74) Representative: **Vossius & Partner**
**Siebertstrasse 4 P.O. Box 86 07 67**
**W-8000 München 86(DE)**

**EP 0 278 505 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to compositions and methods for maintaining the integrity of the hair.

The hair's outer surface, the cuticle, is composed of cells that are held together tightly by a mixture of lipids and proteins. Bleaching, exposure to ultraviolet light, and permanent wave treatments weaken the linkages between the cuticle's cells. Once these linkages are weakened, everyday washing, even with mild shampoos, extracts proteins, amino acids and other essential ingredients from the hair, thereby even further weakening the linkages. If left unchecked, that can lead to excessive dryness, brittleness, split ends, and lack of manageability of the hair.

The present invention is based on the discovery that certain cholesterol derivatives when combined with either certain ceramides or certain glycoceramides inhibits substantially the extraction of proteins and amino acids from the hair. Such extractions occurs, for example, when the hair is exposed to shampoos.

More particularly, the present invention relates to a hair protection composition for application to the hair comprising:

a) at least one ceramide or glycoceramide having the formula

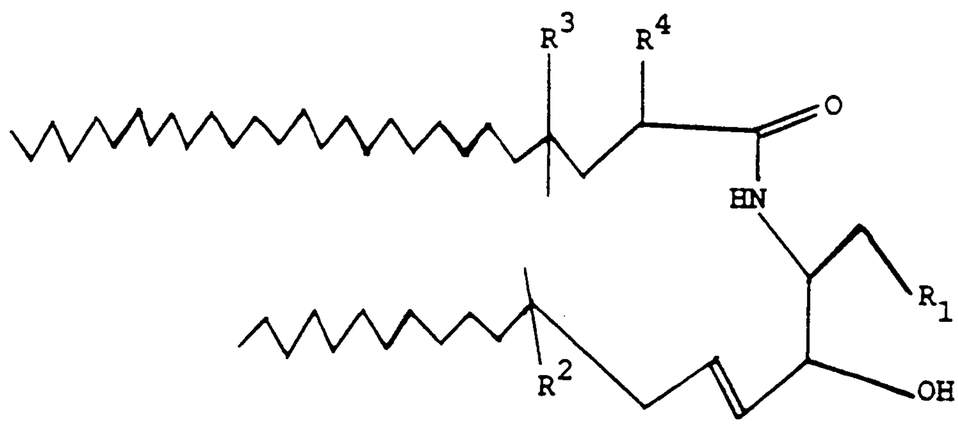

wherein $R^1$ is -OH, O-glucose$_n$ wherein n is an integer from 1 to 4, or O-galactose$_m$ wherein m is an integer from 1 to 8, $R^2$ is $C_{11}$ to $C_{14}$ alkyl, $R^3$ is $C_{12}$ to $C_{24}$ alkyl, and $R^4$ is hydrogen or hydroxy; and

b) at least one cholesterol derivative of the formula

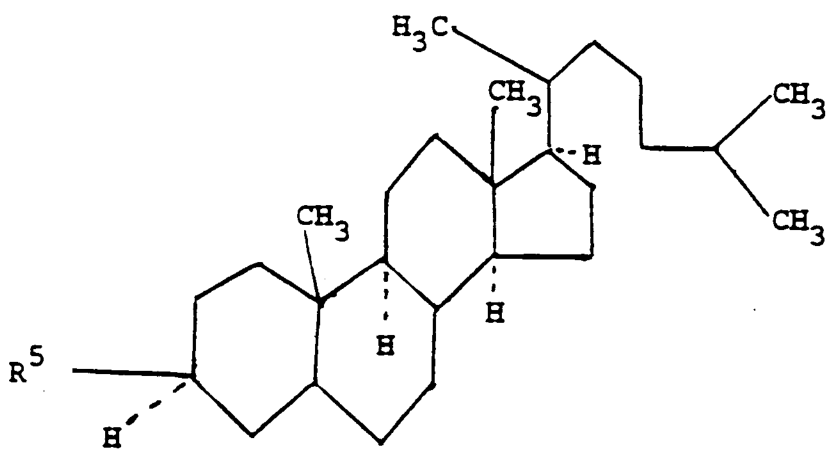

wherein $R^5$ is

$HOO_2SO$-, $CH_3COO$- or $HOOC(CH_2)_pO$- wherein p is an integer from 9 to 17, and

c) a cosmetically acceptable vehicle.

When the foregoing constituents are combined all or part of component (a) may form a weak complex with component (b). It should be understood, therefore, that the composition of the present invention includes both mixtures of the foregoing constituents as well as complexes formed from the constituents.

The composition of the invention comprises a ceramide or glycoceramide in combination with a

cholesterol derivative incorporated into any cosmetically acceptable vehicle adapted for application to the hair, such as a shampoo or a conditioner. Such vehicles, of course, should not be irritating or otherwise harmful to the skin and the resulting product should, preferably, have a pleasant odor or be odorless.

Shampoo formulations of the present invention generally will contain an effective amount of ceramide or glycoceramide, an effective amount of cholesterol derivative, water, and a cleaning agent (e.g., a surfactant and/or a detergent) and, optionally, a thickening agent and/or fragrance and/or at least one preservative.

Hair conditioner formulations of the present invention generally will contain an effective amount of ceramide or glycoceramide, an effective amount of cholesterol derivative, and water. Preferably, such compositions will also contain an emulsifier system, at least one conditioning agent (which provides surface slip), and a preservative and, optionally, a fragrance, a sunscreen, or both.

Cleaning agents that may be used in the compositions of the present invention include, but are not limited to, sodium lauryl sulfate, ammonium lauryl sulfate, sodium lauryl sarcosinate, Triton-X-100® (Rohm and Haas Co.), and triethanolamine lauryl sulfate.

Thickening agents that may be used in the compositions of the present invention include, but are not limited to, hydroxypropyl methyl cellulose, carbopols (manufactured by B. F. Goodrich Co.), magnesium-aluminum silicates (e.g., Veegum®, manufactured by R. T. Vanderbilt Company, Inc.) and lauramide diethanolamine.

Any fragrances compatible with the particular vehicle utilized may be used in the compositions of the present invention.

Preservatives that may be used in the compositions of the present invention include, but are not limited to, imidazolinyl urea (available as Germall® 115, manufactured by Sutton Laboratories, Inc.), phenoxyethanol, methyl paraben, propyl paraben, butyl paraben, and combinations of two or three of the aforementioned parabens.

Emulsifiers that may be used in the compositions of the present invention include, but are not limited to, 10 parts by weight of beeswax and about 0.1 to 1.0 parts by weight of borax), and compositions consisting essentially of stearic acid and triethanolamine (e.g., about 1 to 15 parts by weight of stearic acid and about 0.1 to 2.0 parts by weight of triethanolamine).

Conditioning agents that may be used in the compositions of the present invention include, but are not limited to, hydrolyzed animal protein, panthenol, Merquat 500® (Merck & Co., Inc.), stearalkonium chloride, and Polymer JR® (Dow Chemical Co.).

Sunscreens that may be used in the compositions of the present invention include any recognized and approved sunscreen at appropriate levels.

The compositions of the present invention contain an effective amount of ceramide or glycoceramide and an effective amount of cholesterol derivative, i.e., amounts that are effective to provide a protective or repair effect to the hair. The protective effect can be measured by measuring the amount of protein and amino acids that may be extracted from the hair by shampooing with a mild surfactant (e.g., 5% sodium lauryl sulfate). The greater the protective effect, the more difficult it is to remove protein and amino acids from the hair with such a surfactant.

Although even a relatively low concentration of ceramide or glycoceramide and a relatively low concentration of cholesterol derivative are effective in the compositions of the present invention, the concentration of ceramide or glycoceramide in a composition of the present invention should desirably be at least about 0.1% percent by weight of the composition and the concentration of cholesterol derivative should desirably be at least about 0.1% percent by weight of the composition. Ease of formulation, ease of application, and cost factors will determine the maximum desirable concentrations of these materials.

Generally, a preferred concentration of ceramide or glycoceramide in the compositions of our invention is from about 0.1% percent by weight to about 20% percent by weight of the composition. More preferably, the concentration ranges from about 1% to about 10% percent by weight of the composition.

Generally, a preferred concentration of cholesterol derivative in the compositions of our invention is from about 0.1% percent by weight to about 10% percent by weight of the composition. More preferably, the concentration ranges from about 0.5% to about 5% percent by weight of the composition.

Preferably, the ratio of ceramide or glycoceramide to cholesterol derivative will range from about 10 to 1 to about 1 to 1, more preferably from about 4 to 1 to about 2 to 1.

The frequency of application of the compositions of the present invention to the hair will depend on such factors as the condition of the hair, the age of the individual to whom the composition is to be applied and the vehicle used. Generally, the compositions of the present invention will be applied from one to several times per week (e.g., as a shampoo or conditioner).

Ceramides and glycoceramides are generally available as partially pure lipids derived from porcine skin, bovine brain, red blood cells, or plant extracts. A preferred source is bovine brain extract (available from

Pentapharm, Inc. Basel Switzerland).

Cholesterol derivatives are generally synthetic in nature and are available as ultra pure materials. Sigma Chemical Co., Fisher Scientific and American Scientific Supply Inc. supply suitable materials.

The following Examples illustrate various compositions of the present invention.

## EXAMPLES

The following formulation are prepared by mixing together the ingredients listed below:

### Example 1

SHAMPOO (Low Conditioning)

| Parts By Weight | Ingredient |
|---|---|
| 38.525 | Deionized Water |
| 33.800 | Sodium Lauryl Sulfate |
| 15.000 | Henna |
| 0.500 | Methyl Paraben |
| 0.300 | Propyl Paraben |
| 0.500 | Imidazolidinyl Urea |
| 0.100 | Disodium EDTA |
| 5.000 | Sodium Laureth Sulfate |
| 2.000 | Lauramide DEA |
| 2.000 | Glycol Stearate |
| 0.425 | Citric Acid Anhydrous |
| 0.100 | Sodium Chloride |
| 0.400 | Ethoxydiglycol |
| 0.100 | F D & C Blue #1 (1.0% Aqueous Solution) |
| 0.050 | F D & C Yellow #5 (1.0% Aqueous Solution) |
| 1.000 | Ceramide (Sigma Chemical Co. C2137) |
| 0.100 | Cholesterol Sulfate (Sigma Chemical Co. 9523) |
| 0.100 | Galactosyl Ceramide (Pentapharm, Inc.) |

## Example 2

### PROTECTIVE SHAMPOO (Conditioning)

| Parts By Weight | Ingredient |
|---|---|
| 51.89 | Deionized Water |
| 30.00 | Ammonium Lauryl Sulfate |
| 4.00 | Ammonium Laureth Sulfate |
| 1.00 | Quaterium 24 |
| 0.15 | Citric Acid |
| 1.35 | Hydrolysed Animal Protein (Croda Inc.) |
| 3.00 | Lauramid DEA |
| 0.50 | Isostearimide DEA |
| 0.50 | Steareth 20 |
| 0.20 | Disodium EDTA |
| 0.50 | Methyl Paraben |
| 0.30 | Propyl Paraben |
| 0.50 | Imidazolidinyl Urea |
| 0.50 | Castor Oil |
| 0.50 | Lecithin |
| 1.00 | PEG-40 Lanolin |
| 2.00 | Igepal |
| 0.10 | Soybean Oil |
| 0.10 | Caprylic Triglyceride |
| 0.40 | Ethoxydiglycol |
| 0.01 | Chamomile |
| 0.40 | Disodium Copper EDTA |
| 0.20 | Cholesterol Sulfate |

| Parts Weight | Ingredient |
|---|---|
| 0.20 | Cholesterol Acetate (Sigma Chemical Co. C8628) |
| 0.70 | Galactosyl Ceramide |

## Example 3

### HAIR CONDITIONER

| Parts By Weight | Ingredient |
|---|---|
| 91.95 | Water |
| 0.50 | Propylene Glycol |
| 0.20 | Methyl Paraben |
| 0.05 | Propyl Paraben |
| 1.40 | Stearalkonium Chloride |
| 3.00 | Stearyl Alcohol |
| | Cetearyl Alcohol |
| | Steareth 20 |
| | Glycol Distearate |
| [illegible] | Dimethicone |
| 0.35 | F D & C Green #3 |
| 0.15 | F D & C Yellow #5 |
| 0.15 | F D & C Yellow #6 |
| 0.10 | Cholesterol Acetate |
| 0.30 | Galactosyl Ceramide |

## Claims

**1.** A hair protection composition comprising

a) at least one ceramide or glycoceramide having the formula

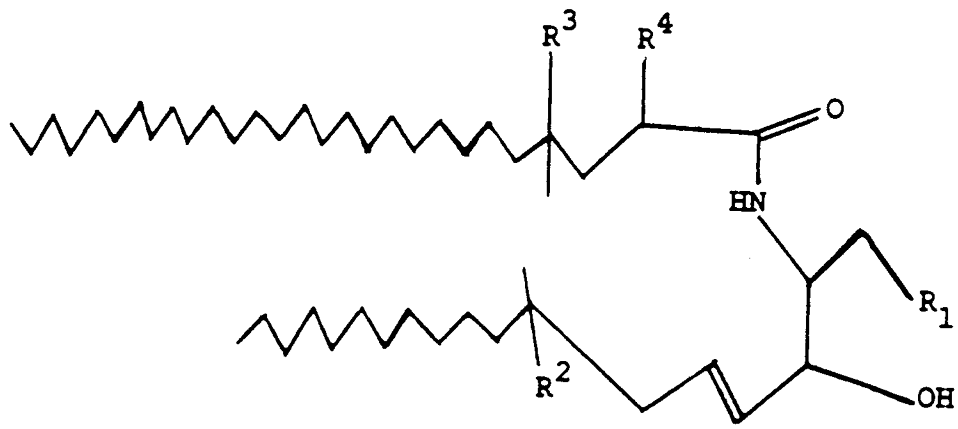

wherein $R^1$ is -OH, O-glucose$_n$ wherein n is an integer from 1 to 4, or O-galactose$_m$ wherein m is an integer from 1 to 8, $R^2$ is $C_{11}$ to $C_{14}$ alkyl, $R^3$ is $C_{12}$ to $C_{24}$ alkyl, and $R^4$ is hydrogen or hydroxy; and

b) at least one cholesterol derivative of the formula

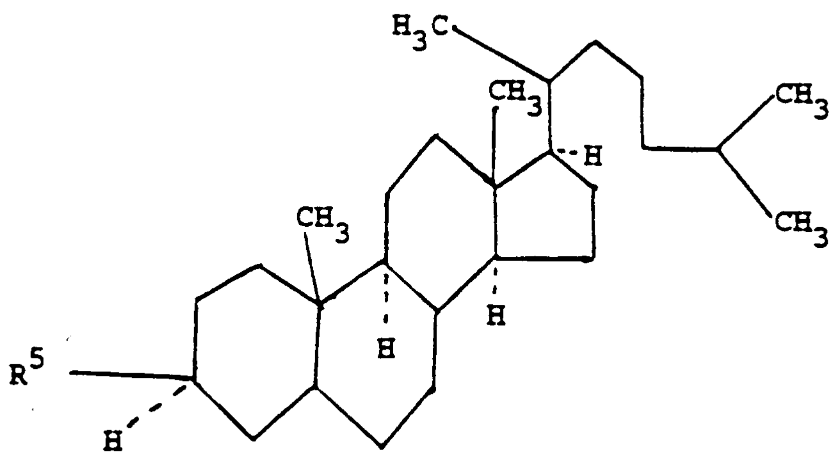

wherein $R^5$ is

$HOO_2SO$-, $CH_3COO$- or $HOOC(CH_2)_pO$- wherein p is an integer from 9 to 17, and

c) a cosmetically acceptable vehicle.

**2.** A composition according to claim 1 additionally comprising water and a cleaning agent.

**3.** A composition according to claim 2 additionally comprising a thickening agent.

**4.** A composition according to any one of claims 2 and 3 additionally comprising a hair conditioning agent.

**5.** A composition according to any one of claims 2 to 4 additionally comprising a sunscreen.

**6.** A composition according to any one of claims 2 to 5 additionally comprising a preservative.

**7.** A composition according to any one of claims 2 to 6 additionally comprising an emulsifier.

**8.** A composition according to claim 1, wherein the concentration of ceramide or glycoceramide is at least about 0.1% by weight of the composition and the concentration of cholesterol derivative is at least about 0.1% by weight of the composition.

**9.** A composition according to claim 1 wherein the concentration of ceramide or glycoceramide is from about 0.1% to about 20% by weight of the composition and the concentration of cholesterol derivative

is from about 0.1% to about 10% by weight of the composition.

**10.** A composition according to claim 1, wherein the concentration of ceramide or glycoceramide is from about 0.1% to about 10% by weight of the composition and the concentration of cholesterol derivative is from about 0.5% to about 5% by weight of the composition.

**11.** A composition according to claim 8, wherein the concentration of ceramide or glycoceramide is at least about 0.2% by weight of the composition and the ratio of ceramide or glycoceramide to cholesterol derivative ranges from about 4 to 1 to about 2 to 1.

**12.** A composition according to any one of claims 1, 8 and 10 wherein the ratio of ceramide or glycoceramide to cholesterol derivative ranges from about 10 to 1 to about 1 to 1.

**13.** A composition according to claim 12, wherein the ratio of ceramide or glycoceramide to cholesterol derivative ranges from about 4 to 1 to about 2 to 1.

**14.** A method of maintaining the integrity of the hair comprising applying to the hair the composition of any one of claims 1 to 13.

**Revendications**

**1.** Composition de protection des cheveux comprenant
a) au moins un céramide ou glycocéramide répondant à la formule :

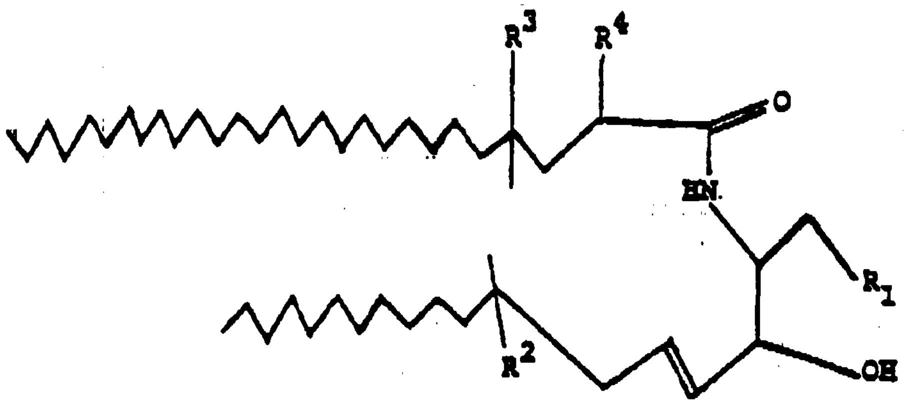

dans laquelle $R^1$ représente -OH, O-glucose$_n$ où n est un nombre entier de 1 à 4, ou O-galactose$_m$ où m est un nombre entier de 1 à 8, $R^2$ représente un alkyle en $C_{11}$ à $C_{14}$, $R^3$ représente un alkyle en $C_{12}$ à $C_{24}$ et $R^4$ représente de l'hydrogène ou de l'hydroxy; et
b) au moins un dérivé de cholestérol de la formule :

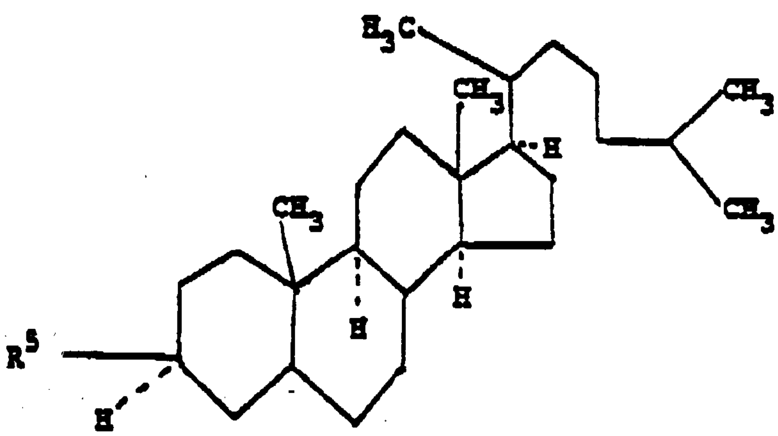

dans laquelle $R^5$ représente $HOO_2SO$-, $CH_3COO$- ou $HOOC(CH_2)_pO$-où p est un nombre entier de 9 à 17, et
c) un véhicule cosmétiquement acceptable.

**2.** Composition suivant la revendication 1, comprenant de plus de l'eau et un agent de nettoyage.

**3.** Composition suivant la revendication 2, comprenant de plus un agent épaississant.

**4.** Composition suivant l'une ou l'autre des revendications 2 et 3, comprenant de plus un agent de conditionnement des cheveux.

**5.** Composition suivant l'une quelconque des revendications 2 à 4, comprenant de plus un écran solaire.

**6.** Composition suivant l'une quelconque des revendications 2 à 5, comprenant de plus un agent de conservation.

**7.** Composition suivant l'une quelconque des revendications 2 à 6, comprenant de plus un émulsifiant.

**8.** Composition suivant la revendication 1, caractérisée en ce que la concentration en céramide ou glycocéramide est d'au moins environ 0,1 % en poids de la composition et la concentration en dérivé de cholestérol est d'au moins environ 0,1 % en poids de la composition.

**9.** Composition suivant la revendication 1, caractérisée en ce que la concentration en céramide ou glycocéramide est d'environ 0,1 % à environ 20 % en poids de la composition et la concentration en dérivé de cholestérol est d'environ 0,1 % à environ 10 % en poids de la composition.

**10.** Composition suivant la revendication 1, caractérisée en ce que la concentration en céramide ou glycocéramide est d'environ 0,1 % à environ 10 % en poids de la composition et la composition en dérivé de cholestérol est d'environ 0,5 % à environ 5 % en poids de la composition.

**11.** Composition suivant la revendication 8, caractérisée en ce que la concentration en céramide ou glycocéramide est d'au moins environ 0,2 % en poids de la composition et le rapport du céramide ou glycocéramide au dérivé de cholestérol est d'environ 4 à 1 à environ 2 à 1.

**12.** Composition suivant l'une quelconque des revendications 1, 8 et 10, caractérisée en ce que le rapport du céramide ou glycocéramide au dérivé de cholestérol est d'environ 10/1 à environ 1/1.

**13.** Composition suivant la revendication 12, caractérisée en ce que le rapport du céramide ou glycocéramide au dérivé de cholestérol est d'environ 4/1 à environ 2/1.

**14.** Procédé permettant de maintenir l'état intact du cheveu, comprenant l'application au cheveu de la composition suivant l'une quelconque des revendications 1 à 13.

**Patentansprüche**

**1.** Haarschutzmittel, umfassend
a) mindestens ein Ceramid oder Glykoceramid der Formel

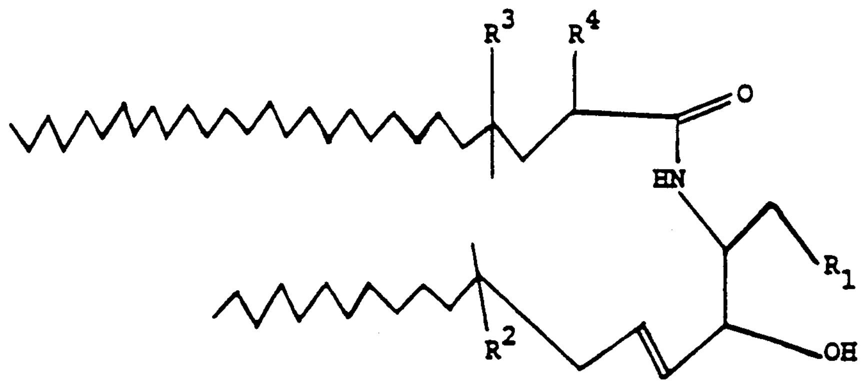

in der $R^1$ eine der Gruppen -OH, O-Glucose$_n$, wobei n eine ganze Zahl von 1 bis 4 ist, oder O-Galactose$_m$, wobei m eine ganze Zahl von 1 bis 8 ist, bedeutet, $R^2$ einen $C_{11}$-$C_{14}$-Alkylrest darstellt, $R^3$ einen $C_{12}$-$C_{24}$-Alkylrest bedeutet und $R^4$ ein Wasserstoffatom oder eine Hydroxylgruppe ist, und
b) mindestens ein Cholesterin-Derivat der Formel

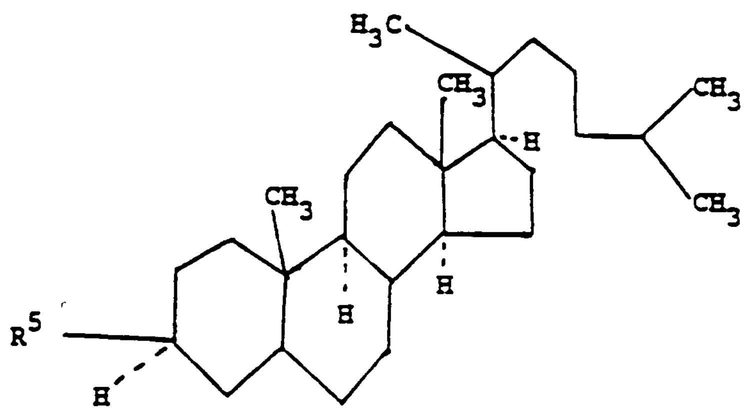

in der $R^5$ eine der Gruppen $HOO_2SO$-, $CH_3COO$- oder $HOOC(CH_2)_pO$- bedeutet, wobei p eine ganze Zahl von 9 bis 17 ist, und
c) einen kosmetisch verträglichen Träger.

**2.** Mittel nach Anspruch 1, zusätzlich umfassend Wasser und ein Reinigungsmittel.

**3.** Mittel nach Anspruch 2, zusätzlich umfassend ein Verdickungsmittel.

**4.** Mittel nach einem der Ansprüche 2 oder 3, zusätzlich umfassend ein Haarpflegemittel.

**5.** Mittel nach einem der Ansprüche 2 bis 4, zusätzlich umfassend einen Sonnenschutz.

**6.** Mittel nach einem der Ansprüche 2 bis 5, zusätzlich umfassend ein Konservierungsmittel.

**7.** Mittel nach einem der Ansprüche 2 bis 6, zusätzlich umfassend einen Emulgator.

**8.** Mittel nach Anspruch 1, in dem die Konzentration des Ceramid oder Glykoceramid mindestens etwa 0,1 Gew.-% des Mittels und die Konzentration des Cholesterin-Derivates mindestens etwa 0,1 Gew.-% des Mittels beträgt.

**9.** Mittel nach Anspruch 1, in dem die Konzentration des Ceramid- oder Glykoceramid etwa 0,1 bis etwa 20 Gew.-% des Mittels und die Konzentration des Cholesterin-Derivats etwa 0,1 bis etwa 10 Gew.-% des Mittels beträgt.

**10.** Mittel nach Anspruch 1, indem die Konzentration des Ceramid oder Glykoceramid etwa 0,1 bis etwa 10 Gew.-% des Mittels und die Konzentration des Cholesterin-Derivats etwa 0,5 bis etwa 5 Gew.-% des Mittels beträgt.

**11.** Mittel nach Anspruch 8, in dem die Konzentration des Ceramid oder Glykoceramid mindestens etwa 0,2 Gew.-% des Mittels beträgt und das Verhältnis von Ceramid oder Glykoceramid zu Cholesterin-Derivat von etwa 4:1 bis etwa 2:1 reicht.

**12.** Mittel nach einem der Ansprüche 1, 8 und 10, in dem das Verhältnis von Ceramid oder Glykoceramid zu Cholesterin-Derivat von etwa 10:1 bis etwa 1:1 reicht.

**13.** Mittel nach Anspruch 12, in dem das Verhältnis von Ceramid oder Glykoceramid zu Cholesterin-Derivat von etwa 4:1 bis etwa 2:1 reicht.

**14.** Verfahren zur Bewahrung der Unverletztheit von Haar, umfassend die Anwendung des Mittels nach einem der Ansprüche 1 bis 13 auf das Haar.